# EUROPEAN PATENT SPECIFICATION

(11) **EP 2 824 182 B1**
(45) Date of publication and mention of the grant of the patent: **02.01.2019**
(21) Application number: 13758671.5
(22) Date of filing: 06.03.2013
(51) Int. Cl.: C12N 15/09, A61K 39/00, A61K 47/42, C12N 5/0783, C12N 5/10, C12Q 1/02, G01N 37/00, G01N 33/53

(54) **METHOD FOR STIMULATING T CELL AND USE THEREOF**
VERFAHREN ZUR STIMULIERUNG VON T-ZELLEN UND VERWENDUNG DAVON
PROCÉDÉ POUR LA STIMULATION DE LYMPHOCYTES T ET SON UTILISATION

(30) Priority: 07.03.2012 JP 2012050018
(43) Date of publication of application: 14.01.2015
(73) Proprietor: National University Corporation University of Toyama, Toyama-shi, Toyama 930-8555 (JP)
(72) Inventor: KISHI, Hiroyuki, Toyama-shi Toyama 930-0194 (JP); MURAGUCHI, Atsushi, Toyama-shi Toyama 930-0194 (JP); HAMANA, Hiroshi, Toyama-shi Toyama 930-0194 (JP); KOBAYASHI, Eiji, Toyama-shi Toyama 930-0194 (JP); OZAWA, Tatsuhiko, Toyama-shi Toyama 930-0194 (JP)
(74) Representative: dompatent von Kreisler Selting Werner - Partnerschaft von Patent- und Rechtsanwälten mbB
(86) International application number: PCT/JP2013/056076
(87) International publication number: WO 2013/133297

(56) References cited:
- EP-A1- 1 566 635
- EP-A1- 2 184 345
- JP-A- 2004 173 681
- JP-A- 2009 034 047
- JIN AISHUN ET AL: "A rapid and efficient single-cell manipulation method for screening antigen-specific antibody-secreting cells from human peripheral blood", NATURE MEDICINE, NATURE PUBLISHING GROUP, NEW YORK, NY, US, vol. 15, no. 9, 1 September 2009 (2009-09-01), pages 1088-1993, XP002573901, ISSN: 1078-8956, DOI: 10.1038/NM.1966 [retrieved on 2009-08-16]
- HELD W. ET AL.: 'Cis interactions of immunoreceptors with MHC and non-MHC ligands.' NAT. REV. IMMUNOL. vol. 8, no. 4, 2008, pages 269 - 278, XP055165920
- SONG Q. ET AL.: 'On-chip activation and subsequent detection of individual antigen- specific T cells.' ANAL. CHEM. vol. 82, no. 2, 2010, pages 473 - 477, XP055165921
- Thomas A Keil ET AL: "Insects as Model Systems in Cell Biology" In: "Electron Microscopy of Model Systems; III. Methods", 1 January 2010 (2010-01-01), Elsevier, XP055424077, ISBN: 978-0-12-381007-6 pages 372-372,
- Thomas A Keil ET AL: "Insects as Model Systems in cell Biology" In: "Electron Microscopy of Model Systems; III. Methods", 1 January 2010 (2010-01-01), Elsevier, XP055424083, ISBN: 978-0-12-381007-6 pages 372-372,

## Description

### Cross Reference of Related Application

### Technical Field

The present invention relates to a method for stimulating and activating a cell with an antigen. The present invention is useful in the fields of life science and medical treatment.

### Background Art

T cells express the T cell receptor (TCR) on the cell surfaces thereof, and they recognize virus-infected cells and cancer cells by using TCR, and induce immune responses. Lymphocytes include B cells besides T cells. B cells also recognize antigens such as viruses by using the antibodies expressed on the cell surfaces thereof as antigen receptors, and the antibodies as the antigen receptors of B cells can bind viruses, proteins, sugar chains, lipids etc. on the surfaces of bacteria in the native forms thereof and thereby enable recognition of them. On the other hand, the antigen receptor of T cell, TCR, cannot bind to viruses or proteins on the surfaces of bacteria in the native forms thereof, and does not enable recognition of them. T cells recognize an antigen through binding of TCR to a peptide produced by decomposition of a virus-derived protein produced in a virus-infected cell, binding to the MHC (major histocompatibility complex) molecule of the cell, and expressed on the cell surface. That is, it is known that, in order to stimulate and activate T cells, an antigen-presenting cell is required. The major antigen-presenting cells include B cells, macrophages, dendritic cells, and so forth, besides virus-infected cells.

The MHC molecules can be roughly classified into two kinds of molecules, i.e., the class I molecules and the class II molecules. The MHC class I molecules present antigens to CD8⁺ T cells (killer T cells), and the MHC class II molecules present antigens to CD4⁺ T cells (helper T cells). The MHC class I molecules are expressed in all the nucleated cells (namely, all the cells except erythrocytes), whereas the MHC class II molecules are expressed in a part of cells, such as B cells, macrophages, and dendritic cells. Moreover, it is known that, in the case of human T cells, the HLA-DR molecule, which is an MHC class II molecule, is expressed in activated CD4⁺ T cells, and HLA-DR is also used as an activation marker (Non-patent document 1).

TCR and MHC molecules exist on T cells, but in a report describing the interactions of the MHC molecules and various immunity-related molecules on the same cells, it is definitely described that TCR and the MHC molecules on the same cells cannot interact (Non-patent document 2). At present, as method for stimulating T cells, when an antigen peptide recognizable by T cells is known, there is generally used a method of preparing antigen-presenting cells such as dendritic cells, and adding an antigen peptide to the antigen-presenting cells to allow binding of the antigen peptide to the MHC molecules on the antigen-presenting cells. Recently, a tetramer prepared by solubilizing an MHC molecule and binding antigen peptides with the solubilized MHC molecules (MHC-peptide tetramer) has been prepared and marketed, and T cells can also be stimulated by using such an MHC-peptide tetramer. Further, although not relating to the method for stimulating T cells, there was reported that a cytokine secreted from a single cell was detected by using a recombinant MHC molecule bound with an antigen peptide for T cells immobilized on a chip (Non-patent documents 3 and 4).

The inventors of the present invention previously developed a microwell array chip on which about 45,000 to 230,000 of microwells having such a size and shape that each well can accommodate only one single cell are regularly arrayed, and demonstrated that antigen-specific B cells can be identified by disposing single B cells in wells of such a chip as mentioned above, stimulating the cells with an antigen, and analyzing change of intracellular Ca²⁺ concentration of the B cells and binding of antigens to antibodies on the surfaces of the B cells at the single cell level. Furthermore, they also demonstrated that antigen-specific antibody-secreting cells can be more conveniently detected by using a microwell array chip of which surface around the wells is coated with antigen (Patent document 1).

### Prior art references

### Patent document

Patent document 1: Japanese Patent Unexamined Publication (Kokai) No. 2009-34047 (Japanese Patent No. 4148367) discloses a cell screening method.

JP 2004 173 681 and EP 1 566 635 A1 disclose microwell array chips for detecting antigen specific lymphocytes.

### Non-patent documents

Non-patent document 1: T Cotner, JM Williams, L Christenson, HM Shapiro, TB Strom, J Strominger, Simultaneous flow cytometric analysis of human T cell activation antigen expression and DNA content, J. Exp. MED., 1983, Vol. 157, 461-472
Non-patent document 2: W Held, RA Mariuzza, Cis interactions of immunoreceptors with MHC and non-MHC ligands, Nature Reviews Immunology, 2008, 8, 269-278
Non-patent document 3: Q Song, Q Han, EM Bradshaw, SC Kent, K Raddassi, B Nilsson, GT Nepom, DA Hafler, JC Love, On-Chip Activation and Subsequent Detection of Individual Antigen-Specific T Cells, Anal. Chem., 2010, 82, 473-477
Non-patent document 4: Q Han, EM Bradshaw, B Nilsson, DA Haflercd, JC Love, Multidimensional analysis of the frequencies and rates of cytokine secretion from single cells by quantitative microengraving, Lab. Chip, 2010, 10, 1391-1400

Aishun Jin et al. in Nature Medicine 15 (2009) 1088-1093 discloses a single cell manipulation method for screening antigen-specific antibody-secreting cells.

### Summary of the Invention

### Object to be Achieved by the Invention

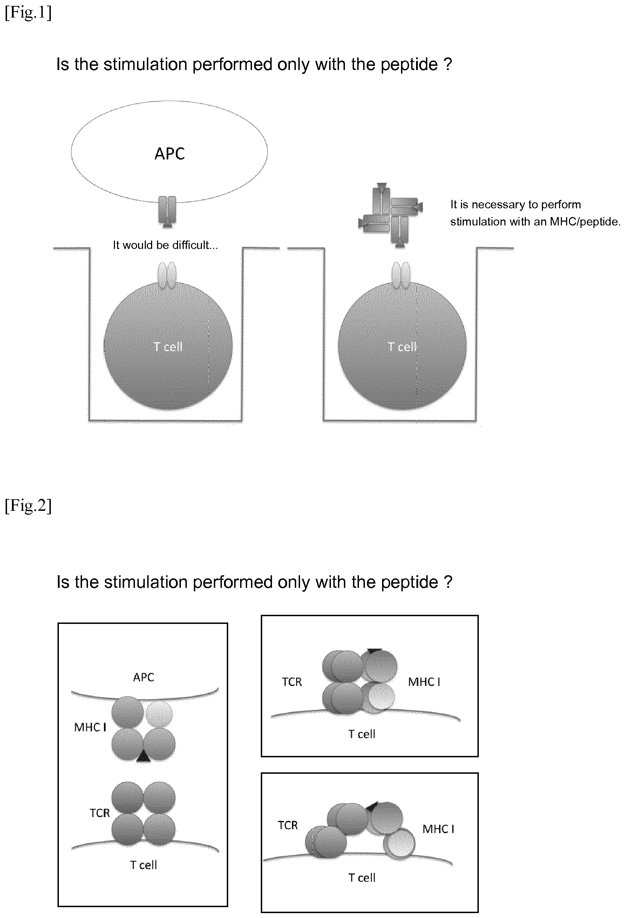

The inventors of the present invention began to develop a method for detecting an antigen-specific T cell by using a microwell array chip. Unlike B cells, T cells are activated through recognition of an antigen peptide bound with an MHC molecule on an antigen-presenting cell by TCR. Therefore, it is considered that, in order to stimulate T cells on a chip, it is necessary to inoculate antigen-presenting cells pulsed with antigen peptides on the chip, but it would be difficult. Therefore, they investigated detection of T cells secreting a cytokine (IL-2, IFN-γ, IL-4 etc.) in an antigen stimulation specific manner 4 to 6 hours after a stimulation with the aforementioned MHC-peptide tetramer ((MHC/p)₄) from T cells arrayed on a microwell array chip. More precisely, it was attempted to detect a cytokine secreted from a T cell stimulated on a microwell array chip and trapped with anti-cytokine antibodies coated on the surface of the chip around the wells, with fluorescence-labeled anti-cytokine antibodies. As a result, a T cell secreting a cytokine in an antigen stimulation specific manner could be detected (Fig. 1).

Although it could be confirmed that an antigen-specific T cell could be detected by using (MHC/p)₄ as described above, (MHC/p)₄ has a problem. Namely, it is difficult to prepare (MHC/p)₄ in general laboratories, and it is usually necessary to purchase and use marketed (MHC/p)₄. However, types of currently marketed (MHC/p)₄ are limited. On the other hand, there are many known antigen peptides for which (MHC/p)₄ are not available. Although peptide vaccines have been developed and used in clinical treatment of cancers, most of them are not available as (MHC/p)₄.

Therefore, the inventors of the present invention investigated whether an antigen-specific T cell could be stimulated on a chip without using (MHC/p)₄.

### Means for Achieving the Object

The inventors of the present invention paid attention to the fact that MHC molecules are expressed on the cell surfaces of T cells, and considered that if an antigen peptide was bound to the MHC molecules on the T cells, TCR and the MHC-peptide complex might interact with each other on one T cell, and the T cell would be thereby stimulated and activated. Namely, they considered that although an antigen peptide presented on an MHC molecule expressed on APC is usually recognized by using TCR in a T cell (Fig. 2, left, ▲ represents peptide), if the antigen peptide could be given to the MHC molecule on the T cell, the peptide bound with the MHC molecule of the T cell (Fig. 2, upper right), it might be recognized by TCR of the same T cell (Fig. 2, Lower right), and thus the T cell could be activated.

Such a stimulating method has not been implemented so far. This is because, since about 100,000 lymphocytes are usually cultured together in one well when T cells are stimulated, it cannot distinguish whether the production of a cytokine or the like is induced by an interaction of the MHC-peptide and TCR on the same cell or an interaction of the MHC-peptide on a certain cell and TCR on another cell. Fortunately, the inventors of the present invention could use a microwell array chip, and therefore they could create a circumstance where interactions are not allowed for one T cell with another T cell by accommodating one T cell in each microwell. T cells were accommodated by one each per one microwell on the chip, and then an antigen peptide that can bind with the MHC molecule was added. As a result, antigen peptide-specific induction of cytokine secretion could be demonstrated.

The above result indicates that if an antigen peptide is bound with an MHC molecule on a T cell, TCR and the MHC-peptide complex on the same cell interact, and the cell itself, i.e., the T cell, is activated (Fig. 2, lower right). This was clarified for the first time by the experiment using the microwell array chip. Further, the object of such a stimulating and activating method would not be limited to T cells, but it would be applicable to various cells.

On the basis of such findings as mentioned above, the present invention was accomplished. The present invention provides methods as defined in the claims. Further disclosed are:
[1] A method for stimulating a target cell with an antigen peptide, wherein:
   the antigen peptide is supplied to an MHC molecule on cell surface of the target cell to form an MHC molecule-antigen peptide complex,
   the target cell is stimulated through recognition of the MHC molecule-antigen peptide complex by a T cell receptor (TCR) that can recognize the antigen peptide, TCR exists on the cell surface of the target cell, or exists on the same plane as that of the target cell, and the T cell is stimulated by an interaction of TCR and the MHC molecule-antigen peptide complex in the positional relationship of cis in the absence of presentation of the antigen peptide by another antigen-presenting cell (APC).
[2] A method for stimulating a target cell, which comprises:
   the step of supplying an antigen peptide to a T cell having a T cell receptor (TCR) that can recognize the antigen peptide on cell surface to form a complex of a major histocompatibility complex (MHC) molecule on the cell surface of the T cell and the antigen peptide, and wherein
   the T cell is stimulated through recognition of the antigen peptide by TCR as the MHC molecule-antigen peptide complex on the same T cell as that expressing the TCR.
[3] A stimulated cell, which has TCR that can recognize an antigen peptide, and an MHC molecule-antigen peptide complex presenting the antigen peptide on cell surface, and is stimulated through recognition of the antigen peptide as the MHC molecule-antigen peptide complex on the cell surface by TCR.
[4] A method for identifying a T cell, which comprises:
   the step of supplying an antigen peptide to a single subject cell, and detecting whether a substance that is produced when the cell has recognized an antigen is produced from the subject cell or not, and wherein
   when the substance is detected, the subject cell is identified as an intended T cell.
[5] The method according to [4], wherein a microwell array having a plurality of wells having such a size that each well can accommodate only one T cell on one main surface of a substrate is used, and the method is performed for identifying a T cell specific to the antigen peptide from a population of subject cells.
[6] The method according to [5], wherein a microwell array having a coating layer of a substance showing a binding property for at least a part of the substance produced when the T cell has recognized an antigen is used;
   the subject cells are accommodated in at least a part of the wells with a culture medium;
   the coating layer and the wells are immersed in a culture medium containing the antigen peptide to culture the subject cells under a state that substances contained in the culture medium can diffuse from the wells to the coating layer; and
   a labeling substance that can specifically bind with the substance produced when the T cell has recognized an antigen, or a labeling substance that can specifically bind with the coating layer is supplied to the coating layer, and when the substance produced when the T cell binding to the substance of the coating layer has recognized an antigen is detected with the labeling substance, the corresponding subject cell is identified as an antigen-specific T cell.
[7] The method according to any one of [4] to [6], wherein the antigen-specific T cell to be identified is a cell derived from human.
[8] A method for producing an antigen-specific T cell, which comprises the step defined in any one of [4] to [7], and further comprises the step of collecting the identified antigen-specific T cell from the well.
[9] A method for producing an antigen-specific TCR gene, which comprises the steps defined in [8], and further comprises the step of obtaining an antigen-specific TCR gene from the collected T cell.
[10] A method for producing an antigen-specific transgenic T cell, which comprises the steps defined in [9], and further comprises the step of introducing the obtained antigen-specific TCR gene into another T cell to obtain an antigen-specific transgenic T cell.
[11] The production method according to [10], wherein the other T cell is derived from an object with a disease or condition that can be treated by a TCR gene therapy.
[12] The method according to any one of [4] to [7], wherein the antigen peptide is a cancer-related antigen, and a cancer-specific T cell is identified.
[13] The production method according to any one of [8] to [10], wherein the antigen peptide is a cancer-related antigen, and a cancer-specific T cell, a cancer-specific TCR gene, or a cancer-specific transgenic T cell is produced.
[14] The production method according to [13], which is for producing a cancer-specific T cell, a cancer-specific TCR gene, or a cancer-specific transgenic T cell used for a treatment of cancer.
[15] Method for determining effect of cancer peptide vaccine
   The method according to any one of [4] to [7], wherein the antigen peptide is a candidate peptide for cancer peptide vaccine, and the method is performed for determining effect of the candidate peptide.
[16] Analysis of immune response mediated by T cell in infectious disease etc.
   The method according to any one of [4] to [7], wherein the subject cells are derived from a subject with an infectious disease, the antigen peptide is an antigen peptide derived from a pathogen of the infectious disease, and the method is performed for analysis of immune response mediated by T cell.
[17] A method for stimulating a T cell, which comprises:
   the step of supplying an antigen peptide to a T cell having a T cell receptor (TCR) that can recognize the antigen peptide and a major histocompatibility complex (MHC) molecule on cell surface, and wherein the T cell is stimulated through an interaction of TCR and the MHC molecule-antigen peptide complex on the cell surface of the same T cell.
[18] A method for stimulating a cell expressing TCR on cell surface with an antigen peptide, which uses
   a major histocompatibility complex (MHC) molecule on cell surface of a target cell.
[19] A method for stimulating a target cell with an antigen peptide, wherein
   the antigen peptide is supplied to the target cell, and the target T cell is stimulated in the absence of presentation of the antigen peptide by another antigen-presenting cell (APC).

### Effect of the Invention

According to the present invention, an antigen-specific T cell can be activated and detected without using any antigen-presenting cell (APC) nor MHC-peptide tetramer.

At present, researches of the TCR gene therapy of cancer are based on establishing an antigen (cancer)-specific T cell strain, isolating and obtaining a cancer-specific TCR, introducing the obtained TCR gene into a T cell derived from an object to obtain a cancer-specific T cell, and returning such a T cell to the object. In contrast, according to the present invention, an antigen-specific T cell can be identified without establishing such an antigen-specific T cell strain, and without using such a reagent as MHC-peptide tetramer. That is, a cancer-specific T cell can be efficiently and conveniently identified.

The antigen-specific T cell identified by the method of the present invention can be proliferated, and a treatment can be performed with the T cells. Further, by obtaining an antigen-specific TCR gene from the antigen-specific T cell identified by the method of the present invention, TCR gene therapy can be performed.

### Brief Description of the Drawings

[Fig. 1] Activation of T cells with APC or (MHC/p)₄ on microwell array chip: Unlike B cells, T cells are activated through recognition of an antigen peptide bound with an MHC molecule on an antigen-presenting cell by TCR. Therefore, it is considered that, in order to stimulate T cells on a chip, it is necessary to inoculate antigen-presenting cells pulsed with the antigen peptide to the chip, but it would be difficult. Otherwise, T cells on a chip can be stimulated also by using (MHC/p)₄, but it is limited to a case where (MHC/p)₄ is available for a desired peptide.
[Fig. 2] Conceptual diagram of the present invention: T cells usually recognize an antigen peptide (▲) presented on an MHC I molecule expressed on APC by using TCR as shown in the left drawing. However, it is considered that, if a T cell is stimulated by using a peptide on a microwell array chip, the peptide binds to the MHC I molecule on the T cell, the T call recognizes it with TCR, and the T cell is activated as a result, as shown in the right drawings.

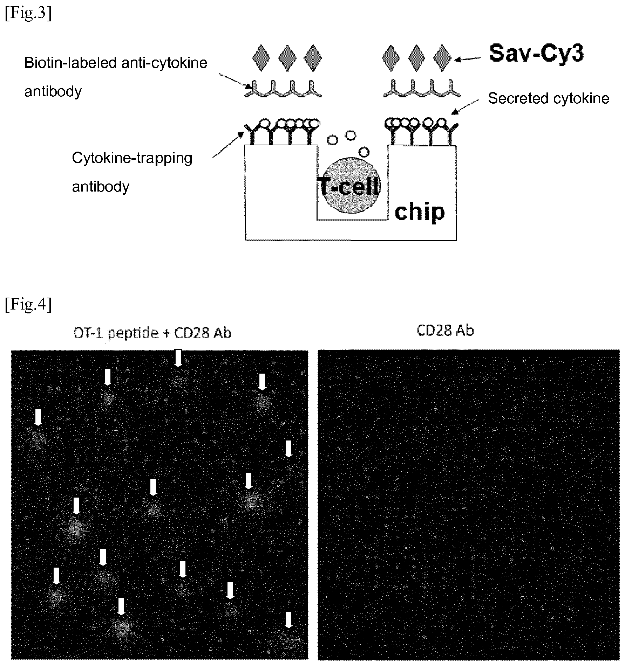

[Fig. 3] Schematic diagram of ISAAC: A cytokine secreted from a T cell is trapped by a cytokine-trapping antibody coated on the chip surface beforehand. The trapped cytokine is detected with a biotin-labeled anti-cytokine antibody and streptavidin (Sav)-Cy3.
[Fig. 4] Detection of secretory cell: Mouse lymphocytes including T cells having TCR that recognizes an ovalbumin (OVA)-derived peptide (OT-1 peptide) were inoculated on a microwell array chip coated with anti-interleukin-2 (IL-2) antibodies, and stimulated with the OT-1 peptide and CD28 antibodies (left) or only CD28 antibodies (right) over 6 hours, and secreted IL-2 was detected by using a biotin-labeled anti-IL-2 antibodies and Sav-Cy3. The cytokine was detected on the chip where the stimulation was performed with the OT-1 peptide and the CD28 antibodies (left), but the cytokine was not secreted when the stimulation was performed only with the CD28 antibodies (right). In the drawings, the small dots represent the cells, and doughnut- or disk-shaped signals indicated with arrows represent the secreted cytokine.

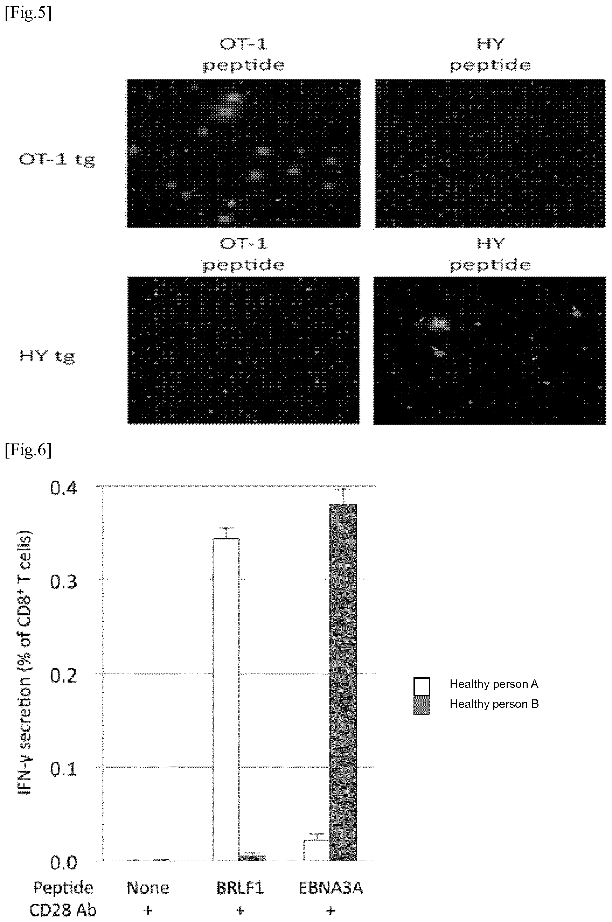

[Fig. 5] Antigen peptide-specific activation of T cell: (Upper drawings) The lymphocytes including OT-1 TCR transgenic mouse-derived T cells produced IL-2, when they were stimulated with the OT-1 peptide as an antigen and CD28 antibodies (upper left), but they did not produce IL-2, when they were stimulated with H-Y peptide, which is not an antigen, and CD28 antibodies (upper right). (Lower drawings) In contrast, the lymphocytes including HY-TCR transgenic mouse-derived T cells produced IL-2, when they were stimulated with the H-Y peptide as an antigen and CD28 antibodies (lower right), but they did not produce IL-2, when they were stimulated with the OT-1 peptide, which is not an antigen, and CD28 antibodies (lower left) (OT-1 peptide = ovalbumin-derived peptide, H-Y peptide = male antigen (H-Y antigen)-derived peptide).
[Fig. 6] Detection of EB virus-derived peptide (BRLF1 peptide, EBNA3A peptide)-specific human T cell: Lymphocytes derived from a healthy person A (white column) or a healthy person B (gray column) were inoculated on a chip, and the cells were stimulated on the chip over 6 hours with only CD28 antibodies, BRLF1 peptide and CD28 antibodies, or EBNA3A peptide and CD28 antibodies, and IFN-γ secreting cells were counted under a fluorescence microscope.

### Modes for Carrying out the Invention

The present invention provides a novel method for stimulating a cell. In the present invention, when a target cell is stimulated with an antigen peptide, a major histocompatibility complex (MHC) molecule of the target cell itself is used, and TCR and the MHC molecule-antigen peptide complex (also referred to as "MHC-peptide complex") are in the positional relationship of cis. For the interaction of TCR and the MHC-peptide complex, it has conventionally been considered that they should be in the positional relationship of trans. In the present invention, the target cell is stimulated in the absence of presentation of the antigen peptide by another antigen-presenting cell (APC).

The present invention can be typically applied to a T cell having TCR and an MHC molecule on the same cell. It is recently known that by introducing the genes of the TCRalpha chain, TCRbeta chain, CD3epsilon chain, CD3gamma chain, CD3delta chain, and zeta chain together into a cell other than T cell, for example, epithelium cells etc., signal-transducible TCR can be expressed on the cell surface (AL Szymczak, CJ Workman, Y Wang, KM Vignali, S Dilioglou, EF Vanin, and DAA Vignali, Correction of multi-gene deficiency in vivo using a single 'self-cleaving' 2A peptide-based retroviral vector, Nature Biotech., 22:589-594, 2004). It is also known that by binding the extracellular moiety of TCR to the zeta chain to prepare a chimeric molecule, a TCR/zeta chain chimeric molecule that can transduce a signal even in the absence of the CD3epsilon chain, CD3gamma chain, and CD3delta chain can be expressed on a cell other than T cell (RA Willemsen, MEM Weijtens, C Ronteltap, Z Eshhar, JW Gratama, P Chames and RLH Bolhuis, Grafting primary human T lymphocytes with cancerspecific chimeric single chain and two chain TCR, Gene Therapy, (2000) 7, 1369-1377; AL Szymczak, CJ Workman, Y Wang, KM Vignali1, S Dilioglou1, EF Vanin, and DAA Vignali, Correction of multi-gene deficiency in vivo using a single 'self-cleaving' 2A peptide-based retroviral vector, Nature Biotechnology, Volume 22, Number 5, May 2004). Furthermore, it is practiced to coat a lipid on a substrate such as ELISA plate, slide glass, and cover glass, and express TCR as a GPI anchor type protein thereon, or express an MHC molecule thereon (A Hashimoto-Tane, T Yokosuka, K Sakata-Sogawa, M Sakuma, C Ishihara, M Tokunaga, and T Saito, Dynein-Driven Transport of T Cell Receptor Microclusters Regulates Immune Synapse Formation and T Cell Activation, Immunity, 34, 919-931, June 24, 2011. It has conventionally been considered that, also in these systems, when a signal is detected, it is necessary to perform stimulation with an MHC-peptide complex on APC, or an MHC-peptide tetramer. However, according to the present invention, by making TCR and the MHC-peptide complex exist on the same cell or the same plane, they can interact with each other.

The stimulation method of the present invention is also a method comprising the step of supplying an antigen peptide to a target cell having a T cell receptor (TCR) that can recognize the antigen peptide and a major histocompatibility complex (MHC) molecule on the cell surface of the target cell, wherein the target cell is stimulated by an interaction of TCR and the MHC-peptide complex on the cell surface of the same cell. Alternatively, it is also a method comprising the step of supplying an antigen peptide to a T cell receptor (TCR) that can recognize the antigen peptide and exists on the same plane as that of a target cell, wherein the cell is stimulated by an interaction of TCR and an MHC-peptide complex in the positional relationship of cis in the absence of presentation of the antigen peptide by another antigen-presenting cell (APC).

In the following descriptions, the present invention may be explained for a T cell having TCR and an MHC molecule on the same cell as an example, and such an explanation can be applied as it is to not only T cell, but also any other cells on which TCR and an MHC molecule can interact in the absence of another ACP cell.

### [Method for stimulating cell]

Specifically, the stimulation method of the present invention can be implemented with the following steps:
(1) the step of supplying an antigen peptide to a T cell having a T cell receptor (TCR) that can recognize the antigen peptide on the cell surface to form a complex of a major histocompatibility complex (MHC) molecule on the cell surface of the T cell and the antigen peptide, and
(2) the step of allowing TCR to recognize the antigen peptide as an MHC molecule-antigen peptide complex on the cell surface of the same T cell.

In the step (1), an antigen peptide is supplied to a T cell. As for the stimulation of T cell, it has been considered that an antigen-presenting cell incorporates a pathogen, decomposes a pathogen protein to form an antigen peptide, binds it to an MHC molecule, and presents it on the cell surface, and an antigen-specific T cell recognizes it with TCR to stimulate the T cell. Since T cells are usually cultured and stimulated at a density of about 10⁶ cells/mL, whether activation of a T cell is induced with the antigen peptide binding to an MHC molecule present on the same T cell or with the antigen peptide binding to an MHC molecule present on another cell cannot be determined. However, according to the investigation of the inventors of the present invention, it was found that, when an antigen peptide was directly added to an antigen-specific T cell, the peptide bound to MHC on the T cell, and the T cell itself was directly stimulated. Such a finding is provided for the first time by the present invention.

An example of T cell that can be stimulated by the method of the present invention is CD8⁺ T cell (CD8-positive T cell, killer T cell). An MHC class I molecule presents an antigen to the CD8⁺ T cell, and is expressed in all the nucleated cells, and an MHC class I molecule is expressed also in T cell.

Another example of T cell that can be stimulated by the method of the present invention is human CD4⁺ T cell (CD4-positive T cell, helper T cell). An MHC class II molecule presents an antigen to the CD4⁺ T cell, and is usually expressed in a part of cells such as B cell, macrophage, and dendritic cell. However, it is known that, in the case of human T cell, the HLA-DR molecule, which is an MHC class II molecule, is expressed in the activated CD4⁺ T cell, and HLA-DR is used also as an activation marker (Non-patent document 1 mentioned above). Therefore, it can be expected that by adding a peptide that binds to the HLA-DR molecule to a CD4+ T cell, a CD4+ T cell specific to the peptide is stimulated.

At the time of the stimulation, a stimulation enhancer may be given simultaneously with the antigen peptide. When a CD8+ T cell is used, CD28 antibodies can be supplied simultaneously, and 4-1BB and OX40 (Cytokine & Growth Factor Reviews, 14 (2003) 265-273) can also be supplied simultaneously.

By the method of the present invention, a T cell derived from any of various mammals can be stimulated. For example, a T cell derived from mouse or human can be stimulated.

Completion of the step (2) can be confirmed by various methods. For example, if an activated T cell explained below is induced after an antigen peptide is supplied in the absence of presentation of the antigen peptide by an antigen-presenting cell (APC) other than the target T cell, more precisely, after the antigen peptide is supplied to a single target T cell, it can be judged that the step (2) has been completed.

### [Stimulated T cell]

The present invention provides a stimulated T cell stimulated in a specific manner. The stimulated T cell of the present invention has TCR that can recognize an antigen peptide and an MHC molecule-antigen peptide complex presenting the antigen peptide on the cell surface, and has been stimulated by making TCR recognize the antigen peptide as the MHC molecule-antigen peptide complex on the cell surface. Such a stimulated T cell is isolated and provided for the first time by the present invention.

Whether a cell is the stimulated T cell or not can be confirmed by determining presence or absence of production of a substance that is produced when the T cell has recognized an antigen. Such a substance is well known to those skilled in the art. For example, whether a CD8⁺ T cell is a stimulated cell or not can be judged on the basis of the presence or absence of production of IL-2 or TNF-γ. For the specific conditions and procedures, the descriptions of the examples of this specification can be referred to.

### [Method for detecting and identifying antigen-specific T cell]

The present invention provides a method for identifying an antigen-specific T cell as
defined in claim 1.

This method specifically includes the following steps:
(3) the step of supplying an antigen peptide to a single subject cell, and
(4) the step of detecting whether a substance that is produced when a T cell has recognized an antigen is produced from the subject cell or not.

When the substance is detected, the subject cell is identified as an antigen-specific T cell.

When the term "single" is used for a state of cell in the present invention, it means that the cell is in an environment not containing any other cells, unless especially indicated.

This method of the present invention can be performed by using a microwell array having a plurality of wells having such a size that each well can accommodate only one cell on one main surface of a substrate. As for the method for using such a microwell array, the previous patent application of the inventors of the present invention (Patent document 1 mentioned above) can be referred to.

In the case of using such a microwell array, according to a preferred embodiment, there is used a microwell array having a coating layer of a substance showing a binding property for at least a part of a substance produced when the T cell has recognized an antigen on at least a part of the main surface of the microwell array, and the antigen peptide is supplied to the subject cells accommodated in at least a part of the wells.

A microwell array usable in the present invention can be appropriately designed by those skilled in the art with reference to Patent document 1 mentioned above. It is typically such a microwell array as described below.

The microwell array has a plurality of wells on one main surface of a substrate, and the wells have such a size that each well can accommodate only one cell. A plurality of the microwells are horizontally and vertically arrayed with the same interval. The microwells have, for example, a cylindrical shape or a hexagonal pillar shape. In the case of cylindrical shape, it may have a diameter of, for example, 3 to 100 µm, preferably 4 to 15 µm. Further, it may have a depth of, for example, 3 to 100 µm, preferably 4 to 40 µm. Although the number of the microwells of one microwell array chip is not particularly limited, it may be, for example, in the range of 2,000 to 1,000,000 per cm², in view of the fact that the occurrence frequency of antigen-specific lymphocyte is from 1 to as high as about 500 per 10⁵ cells. Further, the microwell array has a coating layer of a substance showing a binding property for at least a part of a substance produced by at least a part of the cells accommodated in the wells on at least a part of the main surface at least in the circumference of the wells. The material of the microwell array may be, for example, silicon, and may be subjected to a surface treatment.

In the present invention, the cells accommodated in the wells (subject cells) consist of a population of cells including an antigen-specific T cell (target T cell). The means for obtaining a population of lymphocytes or a population of T cells from a living body are well known to those skilled in the art. A population of T cells may be further subjected to separation and purification. Although T cells obtained from a living body usually consist of a heterogenous cell population, and include a plurality of subsets, and methods for separating subsets of T cells such as CD8⁺ T cells and CD4⁺ T cells are also well known to those skilled in the art.

When a cell population is inoculated on a microwell array chip, lymphocytes can be used in the form of a suspension in an appropriate culture medium having a density of about 0.5 to 10 x 10⁶ cells/mL.

In the present invention, as the substance showing a binding property (binding substance) for at least a part of a substance that is produced when the T cell has recognized an antigen (producing substance), an antibody against the producing substance can be used. For detection of the presence or absence of the producing substance, a substance that specifically binds to the producing substance or a substance that specifically binds to the binding substance may be used. A typical example of the substance used for the detection is an antibody.

A typical example of the substance that is produced when the T cell has recognized an antigen is a cytokine, and in this case, the substance showing a binding property for the cytokine may be an anti-cytokine antibody or a cytokine receptor. The presence or absence of a cytokine can be detected by using an antibody or cytokine receptor for the cytokine, or an antibody against the substance used as the binding substance.

A typical example of the labeling of the substance for detecting the presence or absence of the producing substance is biotinylation, and if biotinylation is performed, further addition of a fluorescent substance enables detection by various methods. The labeling can be performed by using a known method.

The coating layer of a binding substance can be formed according to the method described in Patent document 1 mentioned above. When the coating is formed, a higher concentration of the binding substance compared with that for the coating used in usual ELISA or the like (1 µg/mL or lower for a typical case of coating with antibodies) may be preferred. When a microwell array is used, it is necessary to trap the producing substance secreted from the well at the circumference of the well. If the amount of the coated antibodies is small, the cytokine does not remain in the circumference of the well, but it diffuses widely, and thus it may become impossible to determine from which well the substance has been secreted. By using a larger amount of the binding substance for coating, substantially all the producing substance diffusing from a well can be trapped in the very vicinity of the well. Therefore, from the cell of which well the producing substance has been produced can be definitely identified, and the intended cell can be definitely identified.

For example, when a CD8⁺ T cell is used as the subject cell, and an anti-IL-2 antibody or anti-INFγ antibody is coated on the surface of the circumference of the wells, the concentration can be 5 µg/mL or higher.

The method for detecting and identifying a T cell of the present invention can be performed specifically with the following steps of:
(5) accommodating the subject cells in at least a part of the wells with a culture medium,
(6) immersing the coating layer and the wells in a culture medium containing the antigen peptide to culture the subject cells under a state that substances contained in the culture medium can diffuse from the wells to the coating layer; and
(7) supplying a labeling substance that can specifically bind with a substance produced when the T cell has recognized an antigen, or a labeling substance that can specifically bind with the coating layer to the coating layer, and when the substance produced when the T cell has recognized an antigen is detected with the labeling substance, identifying the subject cell as an antigen-specific T cell.

### Step (5)

A subject cell population is accommodated in at least a part of the wells of the microwell array. It is preferable to wash the wells and the circumference thereof of the microwell array to fully remove impurities adhering to the surface when the coating layer of the binding substance is formed, before the cells are accommodated, in order to perform the detection with sufficient accuracy. The cells are accommodated in the wells with a culture medium. The cell population to be accommodated in the wells can be obtained by using a known method.

### Step (6)

The coating layer and the wells are immersed in a culture medium containing the antigen peptide to culture the cells under a state that substances contained in the culture medium can diffuse from the wells to the coating layer. If the cell is an antigen-specific T cell for the antigen peptide, the substance is produced during the culture, and the produced substance is released into the culture medium and diffuses from the well to the coating layer at the circumference of the well. The producing substance that has diffused and reached the coating layer binds to the binding substance constituting the coating layer. Culture conditions can be appropriately determined, and culture time can be appropriately determined so that the amount of the producing substance binding with the binding substance constituting the coating layer comes to be a detectable level. In the coating layer around the well in which the accommodated cell does not produce the substance, the binding of the producing substance and the binding substance does not occur. If the culture time is unduly long, the producing substance diffuses too much widely, and it may become difficult to identify the well in which the cell that produces the producing substance is accommodated. Therefore, it is preferred that the culture time is appropriately determined to be within such a range that the well in which the cell that produces the producing substance is accommodated can be easily identified.

### Step (7)

After completion of the aforementioned culture, and the culture medium is arbitrarily removed, a labeling substance that specifically binds to the substance produced by the target T cell included in the subject cells is supplied to the coating layer. The substance produced by the target T cell diffuses during the culture, and binds to the binding substance constituting the coating layer, and if the aforementioned labeling substance is supplied at this stage, the labeling substance binds to the producing substance bound to the coating layer, or the coating layer not blocked with the producing substance binding to the coating layer. In the former case, the labeling substance has a binding property for the producing substance, and in the latter case, the labeling substance has a binding property for the binding substance constituting the coating layer, not for the producing substance.

It is preferable to remove the culture medium before the labeling substance is supplied. Depending on the combination of the cell and the binding substance, even if the labeling substance is supplied to the coating layer without removing the culture medium, the detection may be performed without any problem.

Then, by detecting the substance produced by the target T cell binding to the substance of the coating layer using the labeling substance, the target T cell is identified. The labeling substance is bound with the producing substance binding to the coating layer, and by detecting the labeling substance there, the cell that produces the producing substance binding to the coating layer (target T cell) can be identified.

### [Use of method of the present invention]

The T cell identified by the present invention can be collected, or cell components and information relevant to the antigen-specific TCR (for example, nucleic acids, proteins, nucleotide sequences, and amino acid sequences) can be obtained from the obtained T cell, and they can be used for researches relating to T cells, or test, diagnosis, and treatment of a disease or condition relating to T cells.

More specifically, the followings are provided by the present invention.
(8) A simple method for producing an antigen-specific T cell.
(9) A method for producing an antigen-specific TCR gene comprising the step of obtaining an antigen-specific TCR gene from the collected T cell.
(10) A method for producing an antigen-specific transgenic T cell further comprising the step of introducing the obtained antigen-specific TCR gene into another T cell to obtain an antigen-specific transgenic T cell.

The step of obtaining an antigen-specific TCR gene from the T cell is specifically, for example, a step of isolating mRNA of TCR from the antigen-specific T cell, and obtaining cDNA therefrom by reverse transcription.

In the present invention, various antigen peptides can be used, and various antigen-specific T cells and antigen-specific TCR genes can be thereby obtained.

The term "antigen peptide" used in the present invention refers to a peptide consisting of a part of an antigen protein, having a length enabling presentation to an MHC molecule, for example, a length of 8 to 20 amino acid residues, preferably a length of 8 to 12 amino acid residues, and able to derive an antigen-specific cytotoxic T cell (CTL), a modified peptide thereof having functionally equivalent characteristics, a polytope thereof consisting of two or more of the foregoing peptides bound together, and so forth, unless especially indicated. The modified peptide having functionally equivalent characteristics refers to a modified peptide having the amino acid sequence of the original antigen peptide, but including deletion, substitution and/or addition (including addition of an amino acid residue to the N-terminus or C-terminus of the peptide) of one or several (for example, four or less) amino acid residues, and able to induce TCL in an antigen peptide-specific manner.

A peptide sequence expected to be able to bind with MHC can be searched for by a method well known to those skilled in the art (http://bimas.dcrt.nih.gov/molbio/hla#bind/, or BIMAS HLApeptide binding prediction analysis, J. Immunol., 152,163, 1994). Therefore, those skilled in the art can select a moiety having an MHC binding property from an amino acid sequence of a desired carcinoma antigen protein or virus-derived antigen protein.

In the case of a carcinoma antigen peptide, whether a certain peptide is such an antigen peptide, i.e., whether a certain peptide has an antigen-specific T cell stimulating activity or not, can be determined by a method well known to those skilled in the art, for example, the measurement method described in J. Immunol., 154, p2257, 1995. Specifically, when peripheral blood lymphocytes are isolated from an MHC molecule-positive human, and an object peptide is supplied to them in vitro, if a cytotoxic T cell (CTL) that specifically recognizes the MHC-positive cell pulsed with the peptide is derived, it can be judged that the peptide is an antigen peptide. Whether CTL has been derived or not can be confirmed by measuring presence or absence or amount of IL-2 or IFN-γ, which can be produced by CTL, by an enzyme immunoassay (ELISA).

The antigen peptide (including a modified peptide) used for the present invention can be synthesized by a method similar to the methods used in the usual peptide chemistry. Alternatively, it can be prepared by a method comprising introducing a DNA coding for the antigen peptide into a host, allowing expression of the encoded protein, and purifying a recombinant peptide from the obtained product.

The antigen peptide used for the present invention may be derived from a virus. Further, the antigen peptide used for the present invention may be a cancer-related antigen. Examples of cancer-related antigen include WT1, CEA, AFP, CA19-9, CA125, PSA, CA72-4, SCC, MK-1, MUC-1, p53, HER2, G250, gp-100, MAGE, BAGE, SART, MART, MYCN, BCR-ABL, TRP, LAGE, GAGE, and NY-ESO1.

Application of the method of the present invention to TCR gene therapy is especially expected. By using a disease-related antigen such as cancer-related antigens as the antigen peptide, a T cell specific to the disease-related antigen, and a TCR gene specific to the disease-related antigen can be conveniently and quickly identified and prepared. Further, by introducing the obtained TCR gene into lymphocytes derived from the patient, disease-related antigen-specific T cells effective for treatment of the disease can be obtained. Such a TCR gene therapy can be especially expected as a tailor-made medical treatment for cancer.

The transgenic lymphocytes are cultured into a large number, and then returned to the cancer patient. Since TCR that recognizes the cancer-related antigen is expressed on the lymphocytes, they can recognize cancer cells that present the caner antigen, specifically attack them, and eventually extinguish the cancer cells. The TCR gene therapy has advantages that it does not need to derive lymphocytes that specifically recognize cancer cells presenting a cancer antigen in a body, unlike the treatment with a peptide vaccine, it enables in vitro large scale preparation of lymphocytes having a cancer antigen-specific cytotoxicity, dose of the prepared lymphocytes can be arbitrarily determined, and so forth. The cancer-specific T cell, cancer-specific TCR gene, and method for producing a cancer-specific transgenic T cell of the of the present invention are expected to become more useful, when they are combined with a highly efficient gene transduction means for introducing the TCR gene into lymphocytes derived from a patient, or a means for producing a large number of TCR gene-transduced cells of high quality.

Application of the present invention to a therapy using a cancer peptide vaccine can also be expected. Conventional cancer peptide vaccines are directly administered to a patient (cancer peptide vaccine therapy), or bound to an MHC molecule of an antigen-presenting cell (dendritic cell) and then administered (dendritic cell therapy). In contrast to these therapies, for example, by preparing cancer-specific T cells out of a body, adding a cancer peptide vaccine to the T cells so that the peptide binds to MHC of the T cells, and administering the cells to a patient, a T cell therapy showing a higher curative effect may be provided.

The method of the present invention can be used for test, diagnosis, or treatment of, not only cancer, but also a disease or condition relevant to T cells. The "treatment" referred to in the present invention concerning a disease or condition includes a treatment for reduction of risk of onset, prophylactic treatment, therapeutic treatment, and suppression of advance of the disease or condition.

The method of the present invention can also be used for determining effect of a peptide vaccine. In such a case, by using a candidate of peptide vaccine as the antigen peptide, the aforementioned identification method is performed for determining effect of the candidate peptide, and then by confirming whether T cells are stimulated or not, or confirming degree of the stimulation, effect of the candidate of peptide vaccine can be determined.

Examples of diseases for which application of the present invention can be expected include cancers and infectious diseases, and the cancers include adult cancers and infant cancers, and include gastrointestinal carcinoma, lung cancer, intractable esophageal carcinoma, head and neck cancer, ovarian cancer, multiple myeloma, and so forth. The infectious disease include viral infectious diseases (for example, acquired immunodeficiency syndrome (AIDS), adult T cell leukemia, Ebola hemorrhagic fever, influenza, viral hepatitis, viral meningitis, yellow fever, cold syndrome, rabies, cytomegalovirus infection, severe acute respiratory syndrome (SARS), progressive multifocal leucoencephalopathy, varicella, herpes zoster, hand-foot-and-mouth disease, dengue fever, infectious erythema, infectious mononucleosis, variola, rubella, acute anterior poliomyelitis (polio), measles, pharyngoconjunctival fever (pool fever), Marburg hemorrhagic fever, hantavirus hemorrhagic fever with renal syndrome, Lassa fever, epidemic parotitis, West Nile fever, herpangina, chikungunya hemorrhagic fever, bacterial infection, rickettsial infection, parasitic infection, and prion disease.

The present invention can also be used for analysis of immune response mediated by a T cell. When such an analysis is performed for, for example, analysis of an immune response in infectious disease, the subject cell is a cell derived from a patient of the infectious disease, and the antigen peptide is a pathogen-derived antigen peptide.

The present disclosure also provides a kit used for the method for stimulating a T cell and the method for identifying an antigen-specific T cell provided by the present invention. Such a kit typically comprises a microwell array chip having a coating layer of a substance produced when a T cell has recognized an antigen (producing substance), in order to identify an antigen-specific T cell. Besides the microwell array chip, the kit may comprise a means for separating T cells from a living body, antibody for detection of a producing substance, instruction for implementing the method, instruction describing use and principle of the kit (that is, the kit is for identifying an antigen-specific T cell, TCR on a T cell recognizes an antigen peptide presented by MHC on the same T cell, etc.), and so forth.

### Examples

### Example 1: Detection of antigen-specific mouse T cell

### Preparation of T cells

Lymphocytes of an OT-1 TCR transgenic mouse into which the gene of TCR that recognizes an ovalbumin (OVA)-derived peptide, the OT-1 peptide, was introduced were prepared from the spleen and lymph nodes of the mouse. The lymphocytes were suspended in 10% FCS/RPMI1640 medium at a density of 2.5 x 10⁶ cells/mL, and used for the following experiments.

### Preparation of chip

Anti-IL-2 antibodies (5 µg/mL) were put on a microwell array chip (10 µm in diameter, 126,000 wells), and incubated overnight at room temperature to be bound to the chip surface. On the next day, the chip was washed with phosphate buffered saline (PBS), then PBS containing 0.01% Lipidure BL103 (NOF Corporation) was added to the chip surface, and the chip was placed under reduced pressure so that the air in the microwells was evacuated, and Lipidure was contacted with the chip surface and internal surfaces of the wells to perform blocking (room temperature, 15 minutes or longer). Then, Lipidure on the chip was replaced with a cell culture medium (10% FCS, RPMI1640).

### Addition of cells to chip

The cell culture medium on the chip was removed, 100 µL of the aforementioned cell suspension was added to the chip, and the chip was left standing at room temperature for 5 minutes. The cell suspension was stirred by using a pipette, and left standing for further 5 minutes. The cell suspension was stirred once again, and left standing for further 5 minutes. Finally, stirring of the cell suspension, removal of the cell suspension, and addition of the cell culture medium were repeated 4 or 5 times, and the cells on the chip surface not contained in the wells were removed.

### Detection of cytokine

### Analysis (1)

A chip consisting of the aforementioned chip on which the cells derived from the OT-1 TCR transgenic mouse were inoculated was prepared as described above. Then, the culture medium on the surface of the chip was replaced with the culture medium containing 1 µg/mL of the antigen peptide (OT-1 peptide) and 1 µg/mL of anti-CD28 antibodies, or the culture medium containing only 1 µg/mL of anti-CD28 antibodies, and the chip was incubated in a CO₂ incubator under the conditions of 5% CO₂ and 37°C to stimulate the cells. Six hours after the start of the stimulation, the cell culture medium on the surface of the chip was removed, the chip was washed 3 times with PBS, biotin-labeled anti-IL-2 antibodies was added to the chip, and the chip was incubated at room temperature for 30 minutes. Then, the chip was washed 3 times with PBS, Cy3-labeled streptavidin was added, and the chip was incubated at room temperature for 30 minutes. The chip was washed with PBS 3 times, CellTrace Oregon Green was added, and the chip was incubated at room temperature for 5 minutes to stain the cells. The chip was washed 3 times with PBS, and then fluorescence was observed with a fluorescence microscope (Fig. 3).

The results are shown in Fig. 4. In the photographs, the small dots represent the cells, and doughnut- or disk-shaped signals indicated with arrows represent the secreted cytokine. The cytokine was detected on the chip where the stimulation was performed with the OT-1 peptide and the CD28 antibodies (Fig. 4, left), but the cytokine was not detected when the stimulation was performed only with the CD28 antibodies (Fig. 4, right). That is, it was revealed that the cytokine is specifically secreted by peptide stimulation.

### Example 2: Detection of antigen-specific mouse T cell

### Preparation of T cells

Lymphocytes of an H-Y TCR transgenic mouse into which the gene of TCR that recognizes an H-Y antigen-derived peptide, which is specifically expressed in male, was introduced, and lymphocytes of an OT-1 TCR transgenic mouse into which the gene of TCR that recognizes an ovalbumin (OVA)-derived peptide, the OT-1 peptide, was introduced were prepared from the spleens and lymph nodes of the mice, respectively. The lymphocytes were suspended in 10% FCS/RPMI1640 medium at a density of 2.5 x 10⁶ cells/mL, and used for the following experiments.

### Preparation of chip

The anti-IL-2 antibodies (5 µg/mL) were put on a microwell array chip (10 µm in diameter, 126,000 wells), and incubated overnight at room temperature to be bound to the chip surface. On the next day, the chip was washed with PBS, then PBS containing 0.01% Lipidure BL103 (NOF Corporation) was added to the chip surface, and the chip was placed under reduced pressure so that the air in the microwells was evacuated, and Lipidure was contacted with the chip surface and internal surfaces of the wells to perform blocking (room temperature, 15 minutes or longer). Then, Lipidure on the chip was replaced with a cell culture medium (10% FCS, RPMI1640).

### Addition of cells to chip

The cell culture medium on the chip was removed, 100 µL of the aforementioned cell suspension was added to the chip, and the chip was left standing at room temperature for 5 minutes. The cell suspension was stirred by using a pipette, and left standing for further 5 minutes. The cell suspension was stirred once again, and left standing for further 5 minutes. Finally, stirring of the cell suspension, removal of the cell suspension, and addition of the cell culture medium were repeated 4 or 5 times, and the cells on the chip surface not contained in the wells were removed.

### Detection of cytokine

### Analysis (1)

A chip consisting of the aforementioned chip on which the cells derived from the OT-1 TCR transgenic mouse were inoculated was prepared as described above. Then, the cell culture medium on the surface of the chip was replaced with the culture medium containing 1 µg/mL of the antigen peptide (OT-1 peptide) and 1 µg/mL of anti-CD28 antibodies, or the culture medium containing 1 µg/mL of the non-antigen peptides (HY peptide) and 1 µg/mL of anti-CD28 antibodies, and the chip was incubated in a CO₂ incubator under the conditions of 5% CO₂ and 37°C to stimulate the cells.

Similarly, a chip consisting of the aforementioned chip on which the cells derived from the HY TCR transgenic mouse were inoculated was prepared as described above. Then, the cell culture medium on the surface of the chip was replaced with the culture medium containing 1 µg/mL of the non-antigen peptide (OT-1 peptide) and 1 µg/mL of anti-CD28 antibodies, or the culture medium containing 1 µg/mL of the antigen peptide (HY peptide) and 1 µg/mL of anti-CD28 antibodies, and the chip was incubated in a CO₂ incubator under the conditions of 5% CO₂ and 37°C to stimulate the cells.

Six hours after the start of the stimulation, the cell culture medium on the surface of the chip was removed, the chip was washed 3 times with PBS, the biotin-labeled anti-IL-2 antibodies was added to the chip, and the chip was incubated at room temperature for 30 minutes. Then, the chip was washed 3 times with PBS, Cy3-labeled streptavidin was added, and the chip was incubated at room temperature for 30 minutes. The chip was washed with PBS 3 times, CellTrace Oregon Green was added, and the chip was incubated at room temperature for 5 minutes to stain the cells. The chip was washed 3 times with PBS, and then fluorescence was observed with a fluorescence microscope (Fig. 5). The lymphocytes including OT-1 TCR transgenic mouse-derived T cells produced IL-2, when they were stimulated with the OT-1 peptide as the antigen and CD28 antibodies, but they did not produce IL-2, when they were stimulated with H-Y peptide, which is not an antigen, and CD28 antibodies. In contrast, the lymphocytes including HY-TCR transgenic mouse-derived T cells produced IL-2, when they were stimulated with H-Y peptide as an antigen and CD28 antibodies, but they did not produce IL-2, when they were stimulated with OT-1 peptide, which is not an antigen, and CD28 antibodies. These results indicate that the single T cells were stimulated by the stimulation with the antigen peptide in an antigen-specific manner, and produced the cytokine.

### Example 3: Detection of antigen-specific human T cell

### Preparation of T cells

Most of Japanese are inapparently infected with the Epstein-Barr virus (EB virus). So far, several peptides such as BRLF1 and EBNA3A has been identified as EB virus-derived peptides that bind to the HLA-A24 molecule and serve as a T cell epitope. Peripheral blood was collected from two of healthy persons A and B in a volume of 20 mL each, equal volume of PBS was added to the blood to dilute it 2-fold, and then the diluted blood was layered on Lymphosepar I (Immuno-Biological Laboratories), and centrifuged at 2000 rpm for 30 minutes to separate a lymphocyte fraction. The lymphocytes were suspended in 10% FCS/RPMI1640 medium at a density of 2.5 x 10⁶ cells/mL, and used for the following experiments.

### Preparation of chip

Anti-interferon-y antibodies (5 µg/mL) were put on a microwell array chip (10 µm in diameter, 126,000 wells), and incubated overnight at room temperature to be bound to the chip surface. On the next day, the chip was washed with PBS, then PBS containing 0.01 % Lipidure BL103 (NOF Corporation) was added to the chip surface, and the chip was placed under reduced pressure so that the air in the microwells was evacuated, and Lipidure was contacted with the chip surface and internal surfaces of the wells to perform blocking (room temperature, 15 minutes or longer). Then, Lipidure on the chip was replaced with a cell culture medium (10% FCS, RPMI1640).

### Addition of cells to chip

The cell culture medium on the chip was removed, 100 µL of the aforementioned cell suspension was added to the chip, and the chip was left standing at room temperature for 5 minutes. The cell suspension was stirred by using a pipette, and left standing for further 5 minutes. The cell suspension was stirred once again, and left standing for further 5 minutes. Finally, stirring of the cell suspension, removal of the cell suspension, and addition of the cell culture medium were repeated 4 or 5 times, and the cells on the chip surface not contained in the wells were removed.

### Detection of cytokine

### Analysis (1)

Chips on which the lymphocytes of the healthy persons A and B were inoculated, respectively, were prepared as described above. Then, the cell culture medium on the surface of each chip was replaced with the culture medium containing 1 µg/mL of the BRLF1 peptide and 1 µg/mL of anti-human CD28 antibodies, the culture medium containing 1 µg/mL of the EBNA3A peptide and 1 µg/mL of anti-human CD28 antibodies, or the culture medium containing 1 µg/mL of anti-human CD28 antibodies as a negative control, and the chip was incubated in a CO₂ incubator under the conditions of 5% CO₂ and 37°C to stimulate the cells. Six hours after the start of the stimulation, the cell culture medium on the surface of the chip was removed, the chip was washed 3 times with PBS, biotin-labeled anti-human IFN-γ antibodies were added to the chip, and the chip was incubated at room temperature for 30 minutes. Then, the chip was washed 3 times with PBS, Cy3-labeled streptavidin was added, and the chip was incubated at room temperature for 30 minutes. The chip was washed with PBS 3 times, CellTrace Oregon Green was added, and the chip was incubated at room temperature for 5 minutes to stain the cells. The chip was washed 3 times with PBS, then fluorescence was observed with a fluorescence microscope, and the IFN-γ-secreting cells were counted (Fig. 6). As shown in the graph, cells that mainly reacted with the EB virus peptide BRLF1 and secreted IFN-γ were detected in the lymphocytes of the healthy person A, and cells that mainly reacted with the EB virus peptide EBNA3A and secreted IFN-γ were detected in the lymphocytes of the healthy person B. The lymphocytes were also stained by using the BRLF1/HLA-A24 tetramer and EBNA3A/HLA-A24 tetramer, and analyzed with a flow cytometer (data not shown). The results of the analysis performed by using the aforementioned chips and the results obtained with the flow cytometer showed the same tendency. That is, also in the analysis performed by using the flow cytometer, T cells that reacted with BRLF1 were observed in the sample of the healthy person A, and T cells that reacted with EBNA3A were observed in the sample of the healthy person B. By these results, it was demonstrated that the method of the present invention can be applied to not only mouse T cells, but also human T cells.

### Industrial Applicability

According to the present invention, an antigen-specific T cell can be detected on a chip. This can be used in the field of clinical test, such as determination of effect of a cancer peptide vaccine, or analysis of immune response mediated by T cells in an infectious disease, etc. The present invention can also be utilized for development of kits, reagents, and apparatuses for detection for the foregoing purposes.

## Claims

1. A method for identifying an antigen-specific T cell, which comprises:
supplying an antigen peptide to a single subject T cell, and detecting in the absence of presentation of the antigen peptide by another antigen-presenting cell (APC) and in the absence of MHC-peptide tetramer whether a cytokine is produced by the subject T cell when the T cell recognizes the antigen peptide, and wherein
when the cytokine is detected, the subject T cell is identified as an antigen-specific T cell.

2. The method according to claim 1, wherein a microwell array having a plurality of wells having such a size that each well can accommodate only one T cell on one main surface of a substrate is used, and the method is performed for identifying a T cell specific to the antigen peptide from a population of subject cells.

3. The method according to any one of claims 1 to 2, wherein the antigen-specific T cell to be identified is a cell derived from human.

4. A method for producing an antigen-specific T cell, which comprises the steps defined in claim 1, and further comprises the step of collecting the identified antigen-specific T cell.

5. A method for producing an antigen-specific T cell receptor (TCR) gene, which comprises the steps defined in claim 4, and further comprises the step of obtaining an antigen-specific TCR gene from the collected T cell.

6. A method for producing an antigen-specific transgenic T cell, which comprises the steps defined in claim 5, and further comprises the step of introducing the obtained antigen-specific TCR gene into another T cell to obtain an antigen-specific transgenic T cell.

7. The production method according to claim 6, wherein the other T cell is derived from an object with a disease or condition that can be treated by a TCR gene therapy.

8. The method according to any one of claims 1 to 3, wherein the antigen peptide is a cancer-related antigen, and a cancer-specific T cell is identified.

9. The production method according to any one of claims 4 to 6, wherein the antigen peptide is a cancer-related antigen, and a cancer-specific T cell, a cancer-specific TCR gene, or a cancer-specific transgenic T cell is produced.

10. The production method according to claim 9, which is for producing a cancer-specific T cell, a cancer-specific TCR gene, or a cancer-specific transgenic T cell suitable for a treatment of cancer.

11. The method according to any one of claims 1 to 3, wherein the antigen peptide is a candidate peptide for cancer peptide vaccine, and the method is performed for determining effect of the candidate peptide.

12. The method according to any one of claims 1 to 3, wherein the subject cells are derived from a subject with an infectious disease, the antigen peptide is an antigen peptide derived from a pathogen of the infectious disease, and the method is performed for analysis of immune response mediated by T cell.

13. A method for stimulating a single target T cell with an antigen peptide, wherein:
the antigen peptide is supplied to a major histocompatibility complex (MHC) molecule on cell surface of the target T cell to form an MHC molecule-antigen peptide complex,
the target T cell is stimulated through recognition of the MHC molecule-antigen peptide complex by a TCR that can recognize the antigen peptide, TCR exists on the cell surface of the target T cell, or exists on the same plane as that of the T target cell, and the target T cell is stimulated by an interaction of TCR and the MHC molecule-antigen peptide complex in the positional relationship of cis in the absence of presentation of the antigen peptide by another antigen-presenting cell (APC).

14. A method for stimulating a single target T cell, which comprises:
the step of supplying an antigen peptide to a T cell having a T cell receptor (TCR) that can recognize the antigen peptide on T cell surface to form a complex of an MHC molecule on the cell surface of the T cell and the antigen peptide, and wherein
the T cell is stimulated through recognition of the antigen peptide by TCR as the MHC molecule-antigen peptide complex on the same T cell as that expressing the TCR.

## Patentansprüche

1. Verfahren zum Identifizieren einer antigenspezifischen T-Zelle, umfassend:
Zuführen eines Antigenpeptids zu einer einzigen Probe T-Zelle und Nachweisen in Abwesenheit einer Präsentation des Antigenpeptids durch eine andere antigenpräsentierende Zelle (APC) und in Abwesenheit eines MHC-Peptid-Tetramers, ob von der Probe T-Zelle ein Cytokin produziert wird, wenn die T-Zelle das Antigenpeptid erkennt, wobei dann, wenn das Cytokin nachgewiesen wird, die Probe T-Zelle als antigenspezifische T-Zelle identifiziert ist.

2. Verfahren gemäß Anspruch 1, wobei ein Mikrowell-Array mit einer Vielzahl von Vertiefungen mit einer solchen Größe, dass jede Vertiefung nur eine einzige T-Zelle auf einer Hauptfläche eines Substrats unterbringen kann, verwendet wird und das Verfahren durchgeführt wird, um eine T-Zelle, die spezifisch für das Antigenpeptid ist, aus einer Population von Probe Zellen zu identifizieren.

3. Verfahren gemäß einem der Ansprüche 1 bis 2, wobei die zu identifizierende antigenspezifische T-Zelle eine vom Menschen stammende Zelle ist.

4. Verfahren zum Herstellen einer antigenspezifischen T-Zelle, das die in Anspruch 1 definierten Schritte umfasst und weiterhin den Schritt des Gewinnens der identifizierten antigenspezifischen T-Zelle umfasst.

5. Verfahren zum Herstellen eines Gens für einen antigenspezifischen T-Zell-Rezeptor (TCR), das die in Anspruch 4 definierten Schritte umfasst und weiterhin den Schritt des Gewinnens eines Gens für einen antigenspezifischen TCR aus der gewonnenen T-Zelle umfasst.

6. Verfahren zum Herstellen einer antigenspezifischen transgenen T-Zelle, das die in Anspruch 5 definierten Schritte umfasst und weiterhin den Schritt des Einführens des gewonnenen antigenspezifischen TCR-Gens in eine andere T-Zelle umfasst, wobei man eine antigenspezifische transgene T-Zelle erhält.

7. Herstellungsverfahren gemäß Anspruch 6, wobei die andere T-Zelle von einem Patienten mit einer Krankheit oder einem Zustand, der durch eine TCR-Gentherapie behandelt werden kann, stammt.

8. Verfahren gemäß einem der Ansprüche 1 bis 3, wobei das Antigenpeptid ein mit Krebs zusammenhängendes Antigen ist und eine krebsspezifische T-Zelle identifiziert wird.

9. Herstellungsverfahren gemäß einem der Ansprüche 4 bis 6, wobei das Antigenpeptid ein mit Krebs zusammenhängendes Antigen ist und eine krebsspezifische T-Zelle, ein krebsspezifisches TCR-Gen oder eine krebsspezifische transgene T-Zelle hergestellt wird.

10. Herstellungsverfahren gemäß Anspruch 9, das zur Herstellung einer krebsspezifischen T-Zelle, eines krebsspezifischen TCR-Gens oder einer krebsspezifischen transgenen T-Zelle, die oder das für die Behandlung von Krebs geeignet ist, dient.

11. Verfahren gemäß einem der Ansprüche 1 bis 3, wobei das Antigenpeptid ein Kandidatpeptid für einen Krebs-Peptidimpfstoff ist und das Verfahren durchgeführt wird, um die Wirkung des Kandidatenpeptid zu bestimmen.

12. Verfahren gemäß einem der Ansprüche 1 bis 3, wobei die Probe Zellen von einem Patienten mit einer Infektionskrankheit stammen, das Antigenpeptid ein von einem Erreger der Infektionskrankheit stammendes Antigenpeptid ist und das Verfahren zur Analyse der von der T-Zelle vermittelten Immunantwort durchgeführt wird.

13. Verfahren zum Stimulieren einer einzelnen Target-T-Zelle mit einem Antigenpeptid, wobei:
das Antigenpeptid einem Haupthistokompatibilitätskomplex(MHC)-Molekül auf der Zelloberfläche der Target-T-Zelle zugeführt wird, wobei ein MHC-Molekül-Antigenpeptid-Komplex entsteht;
die Target-T-Zelle durch Erkennung des MHC-Molekül-Antigenpeptid-Komplexes durch einen TCR, der das Antigenpeptid erkennen kann, stimuliert wird, der TCR auf der Zelloberfläche der Target-T-Zelle vorliegt oder auf derselben Ebene wie die Target-T-Zelle vorliegt und die Target-T-Zelle in Abwesenheit der Präsentation des Antigenpeptids durch eine andere antigenpräsentierende Zelle (APC) durch eine Wechselwirkung von TCR und dem MHC-Molekül-Antigenpeptid-Komplex in der cis-Positionsbeziehung stimuliert wird.

14. Verfahren zum Stimulieren einer einzelnen Target-T-Zelle, umfassend:
den Schritt des Zuführens eines Antigenpeptids zu einer T-Zelle, die einen T-Zell-Rezeptor (TCR), der das Antigenpeptid erkennen kann, auf der T-Zell-Oberfläche aufweist, wobei ein Komplex aus einem MHC-Molekül auf der Zelloberfläche der T-Zelle und dem Antigenpeptid entsteht und wobei
die T-Zelle durch Erkennung des Antigenpeptids durch TCR als MHC-Molekül-Antigenpeptid-Komplex auf derselben T-Zelle wie der, die den TCR exprimiert, stimuliert wird.

## Revendications

1. Procédé pour identifier un lymphocyte T spécifique d'un antigène, qui comprend :
la présentation d'un peptide antigénique à un seul lymphocyte T d'un sujet et la détection, en l'absence de présentation du peptide antigénique par une autre cellule présentatrice de l'antigène (CPA) et en l'absence de tétramère CMH/peptide, de la production ou non d'une cytokine par le lymphocyte T du sujet lorsque le lymphocyte T reconnaît le peptide antigénique, et dans lequel
lorsque la cytokine est détectée, le lymphocyte T du sujet est identifié comme un lymphocyte T spécifique de l'antigène.

2. Procédé selon la revendication 1, dans lequel une matrice de micropuits comprenant une pluralité de puits ayant une taille telle que chaque puits ne peut recevoir qu'un lymphocyte T sur une surface principale d'un substrat est utilisée, et le procédé est réalisé pour identifier un lymphocyte T spécifique du peptide antigénique parmi une population de cellules du sujet.

3. Procédé selon l'une quelconque des revendications 1 à 2, dans lequel le lymphocyte T spécifique de l'antigène à identifier est une cellule dérivée de l'être humain.

4. Procédé pour produire un lymphocyte T spécifique d'un antigène, qui comprend les étapes définies à la revendication 1 et comprend en outre l'étape de récupération du lymphocyte T spécifique de l'antigène identifié.

5. Procédé pour produire un gène de récepteur de lymphocyte T (TCR) spécifique d'un antigène, qui comprend les étapes définies à la revendication 4 et comprend en outre l'étape d'obtention d'un gène de TCR spécifique de l'antigène à partir du lymphocyte T récupéré.

6. Procédé pour produire un lymphocyte T transgénique spécifique d'un antigène, qui comprend les étapes définies à la revendication 5 et comprend en outre l'étape d'introduction du gène de TCR spécifique de l'antigène obtenu dans un autre lymphocyte T pour obtenir un lymphocyte T transgénique spécifique de l'antigène.

7. Procédé de production selon la revendication 6, dans lequel l'autre lymphocyte T est dérivé d'un objet ayant une maladie ou une affection qui peut être traitée par une thérapie génique TCR.

8. Procédé selon l'une quelconque des revendications 1 à 3, dans lequel le peptide antigénique est un antigène lié au cancer, et un lymphocyte T spécifique du cancer est identifié.

9. Procédé de production selon l'une quelconque des revendications 4 à 6, dans lequel le peptide antigénique est un antigène lié au cancer, et un lymphocyte T spécifique du cancer, un gène de TCR spécifique du cancer ou un lymphocyte T transgénique spécifique du cancer est produit.

10. Procédé de production selon la revendication 9, qui sert à produire un lymphocyte T spécifique du cancer, un gène de TCR spécifique du cancer ou un lymphocyte T transgénique spécifique du cancer approprié pour un traitement du cancer.

11. Procédé selon l'une quelconque des revendications 1 à 3, dans lequel le peptide antigénique est un peptide candidat pour un vaccin peptidique contre le cancer, et le procédé est réalisé pour déterminer l'effet du peptide candidat.

12. Procédé selon l'une quelconque des revendications 1 à 3, dans lequel les cellules du sujet sont dérivées d'un sujet ayant une maladie infectieuse, le peptide antigénique est un peptide antigénique dérivé d'un agent pathogène de la maladie infectieuse, et le procédé est réalisé pour l'analyse de la réponse immunitaire médiée par les lymphocytes T.

13. Procédé pour stimuler un seul lymphocyte T cible avec un peptide antigénique, dans lequel :
le peptide antigénique est présenté à une molécule du complexe majeur d'histocompatibilité (CMH) à la surface du lymphocyte T cible pour former un complexe molécule du CMH/peptide antigénique,
le lymphocyte T cible est stimulé par la reconnaissance du complexe molécule du CMH/peptide antigénique par un TCR qui peut reconnaître le peptide antigénique, le TCR existe à la surface du lymphocyte T cible ou existe sur le même plan que celui du lymphocyte T cible, et le lymphocyte T cible est stimulé par une interaction du TCR et du complexe molécule du CMH/peptide antigénique dans la relation positionnelle de *cis* en l'absence de présentation du peptide antigénique par une autre cellule présentatrice de l'antigène (CPA).

14. Procédé pour stimuler un seul lymphocyte T cible, qui comprend :
l'étape de présentation d'un peptide antigénique à un lymphocyte T ayant un récepteur de lymphocyte T (TCR) qui peut reconnaître le peptide antigénique à la surface du lymphocyte T pour former un complexe d'une molécule du CMH, à la surface du lymphocyte T, et du peptide antigénique, et dans lequel
le lymphocyte T est stimulé par la reconnaissance du peptide antigénique par le TCR sous la forme du complexe molécule du CMH/peptide antigénique sur le même lymphocyte T que celui exprimant le TCR.
